# NOUVEAU FASCICULE DE BREVET EUROPEEN

(11) **EP 0 850 636 B2**
(45) Date de publication et mention de la décision concernant l'opposition: **20.11.2013**
(45) Mention de la délivrance du brevet: 06.05.1999
(21) Numéro de dépôt: 97402834.2
(22) Date de dépôt: 25.11.1997
(51) Int. Cl.: A61K 8/41

(54) **Composition de teinture d'oxydation des fibres kératiniques et procédé de teinture mettant en oeuvre cette composition**
Oxidationsfärbung von keratinischen Fasern und Färbeverfahren mit dieser Zusammensetzung
Composition for the oxidative dyeing of keratinic fibres and dyeing process using this composition

(30) Priorité: 23.12.1996 FR 9615892
(43) Date de publication de la demande: 01.07.1998
(73) Titulaire: L'Oréal, 75008 Paris (FR)
(72) Inventeur: Rondeau, Christine, 78500 Sartrouville (FR); Cotteret, Jean, 78480 Verneuil-Sur-Seine (FR); De La Mettrie, Roland, 78110 Le Vesinet (FR)
(74) Mandataire: Dossmann, Gérard

(56) Documents cités:
- EP-A- 0 739 622
- EP-A2- 0 714 954
- WO-A1-95/01772
- WO-A1-95/15144
- DE-A1- 2 543 100
- US-A- 4 025 301
- KEITH C.BROWN,JOHN F.CORBETT: 'The role of meta functional benzene in oxidative hair dyeing.II.Reactions with p-Aminophenols', 1979 pages 191 - 211

## Description

La présente invention a pour objet une composition prête à l'emploi pour la teinture d'oxydation des fibres kératiniques, en particulier des fibres kératiniques humaines telles que les cheveux, comprenant, dans un milieu approprié pour la teinture, au moins une base d'oxydation en association avec au moins un coupleur de type méta-aminophénol substitué, au moins un colorant direct cationique et au moins un agent oxydant, ainsi que le procédé de teinture mettant en oeuvre cette composition. Elle concerne également un kit de coloration pour la préparation d'une telle composition prête à l'emploi.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés indoliques comme le 6-hydroxyindole.

La variété des molécules mises en jeu au niveau des bases d'oxydation et des coupleurs, permet l'obtention d'une riche palette de couleurs.

Il est également connu que pour faire encore varier les nuances obtenues et leur donner des reflets, on peut utiliser, en association avec les précurseurs de colorants d'oxydation et les coupleurs, des colorants directs, c'est à dire des substances colorées qui apportent une coloration en l'absence d'agent oxydant

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants directs appartiennent pour leur très grande majorité à la famille des composés nitrés de la série benzénique et ont l'inconvénient, lorsqu'ils sont incorporés dans des compositions tinctoriales, de conduire à des colorations présentant une ténacité insuffisante, en particulier vis-à-vis des shampooings.

Les documents US-A-4 025 301 et EP-A-0 739 622 décrivent des composition de teinture de fibres kératiniques.

La présente invention vise à proposer de nouvelles compositions pour la coloration d'oxydation des fibres kératiniques et en particulier des fibres kératiniques humaines telles que les cheveux qui permettent d'aboutir à des colorations riches en reflets tout en présentant de bonne propriétés de ténacité, en particulier

Ainsi, la demanderesse vient en effet de découvrir qu'il est possible d'obtenir de nouvelles teintures à la fois riches en reflets et tenaces en associant :
- au moins une base d'oxydation,
- au moins un coupleur choisi parmi les dérivés substitués du méta-aminophénol de formule (I) ci-après et leurs sels d'addition avec un acide,
- au moins un colorant direct cationique de formule (II) ci-après, et
- au moins un agent oxydant.

L'invention a donc pour premier objet une composition prête à l'emploi pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation,
- au moins un coupleur choisi parmi les méta-aminophénols de formule (I) suivante et leurs sels d'addition avec un acide : 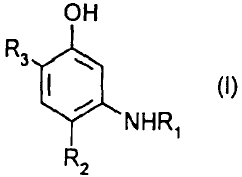 dans laquelle :
   - R₁ représente un atome d'hydrogène, un radical alkyle en C₁-C₄, monohydroxyalkyle en C₁-C₄ ou polyhydroxyalkyle en C₂-C₄,
   - R₂ représente un atome d'hydrogène, un radical alkyle en C₁-C₄, alcoxy en C₁-C₄ ou un atome d'halogène choisi parmi le chlore, le brome ou le fluor,
   - R₃ représente un radical alkyle en C₁-C₄, alcoxy en C₁-C₄, monohydroxyalkyle en C₁-C₄, polyhydroxyalkyle en C₂-C₄, monohydroxyalcoxy en C₁-C₄ ou polyhydroxyalcoxy en C₂-C₄,
   - au moins un colorant direct cationique choisi parmi les composés de formule (II) suivante : 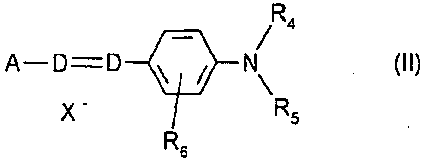
   dans laquelle :
   D représente un atome d'azote ou le groupement -CH,
   R₄ et R₅, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
   R₆ représente un atome d'hydrogène ou d'halogène choisi parmi le chlore, le brome, l'iode et le fluor, un radical alcoxy en C₁-C₄ ou acétyloxy,
   X⁻ représente un anion de préférence choisi parmi le chlorure, le méthyl sulfate et l'acétate,
   A représente un groupement choisi de structures A₁, A₇ et A₁₉ : 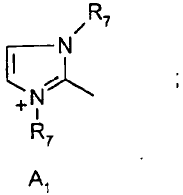 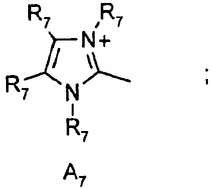 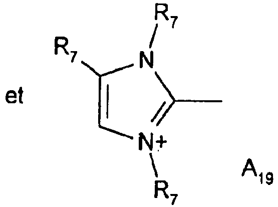
   dans lesquelles R₇ représente un radical alkyle en C₁-C₄ pouvant être substitué par un radical hydroxyle ; et
- au moins un agent oxydant.

Les compositions tinctoriales prêtes à l'emploi conformes à l'invention permettent d'aboutir à des colorations dans des nuances rouges, cuivrées ou violines présentant une bonne résistance aux différents traitements que peuvent subir les cheveux et en particulier vis-à-vis des shampooings.

L'invention a également pour objet un procédé de teinture d'oxydation des fibres kératiniques mettant en oeuvre cette composition tinctoriale prête à l'emploi.

La ou les bases d'oxydation pouvant être utilisées dans les compositions tinctoriales prêtes à l'emploi conformes à invention sont de préférence choisies parmi les paraphénylènediamines, les bis-phénylalkylènediamines, les para-aminophénols, les ortho aminophénols et les bases d'oxydation hétérocycliques.

Selon une forme de réalisation préférée de l'invention, la ou les bases d'oxydation sont choisies parmi les para-phénylènediamines et les para-aminophénols.

Parmi les paraphénylènediamines utilisables à titre de base d'oxydation dans les composition tinctoriales prêtes à l'emploi conformes à l'invention, on peut notamment citer les composés de formule (III) suivante et leurs sels d'addition avec un acide : 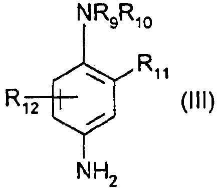 dans laquelle :
R₉ représente un atome d'hydrogène, un radical alkyle en C₁-C₄, monohydroxyalkyle en C₁-C₄, polyhydroxyalkyle en C₂-C₄; phényle, 4'-aminophényle ou alcoxy(C₁-C₄)alkyle en C₁-C₄,
R₁₀ représente un atome d'hydrogène, un radical alkyle en C₁-C₄, monohydroxyalkyle en C₁-C₄ ou polyhydroxyalkyle en C₂-C₄,
R₁₁ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
R₁₂ représente un atome d'hydrogène ou un radical alkyle en C₁-C₄.

Parmi les paraphénylènediamines de formule (III) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 2-β-hydroxyéthyl paraphénylè-nediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-p-acétylaminoéthyloxy paraphénylènediamine, et leurs sels d'addition avec un acide.

Parmi les paraphénylènediamines de formule (III) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, et leurs sels d'addition avec un acide.

Parmi les bis-phénylalkylènediamines utilisables à titre de bases d'oxydation dans les compositions tinctoriales prêtes à l'emploi conformes à l'invention, on peut notamment citer les composés répondant à la formule (IV) suivante, et leurs sels d'addition avec un acide : 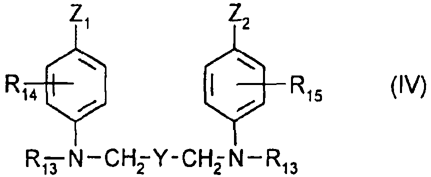 dans laquelle :
Z₁ et Z₂, identiques ou différents, représentent un radical hydroxyle ou NHR₁₆ dans lequel R₁₆ représente un atome d'hydrogène ou un radical alkyle en C₁-C₄,
R₁₃ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₁₄ et R₁₅, identiques ou différents, représentent un atome d'hydrogène ou d'halogène ou un radical alkyle en C₁-C₄,
Y représente un radical pris dans le groupe constitué par les radicaux suivants :
-(CH₂)ₙ ; -(CH₂)ₘ-O-(CH₂)ₘ; -(CH₂)ₘ-CHOH-(CH₂)ₘ et 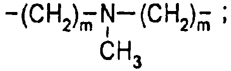 dans lesquels n est un nombre entier compris entre 0 et 8 inclusivement et m est un nombre entier compris entre 0 et 4 inclusivement.

Parmi les bis-phénylalkylènediamines de formules (IV) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-amino-phényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (IV), le N,N'-bis-(β-hydroxyéthyl) N,N'bis-(4'-aminophényl) 1,3-diamino propanol ou l'un de ses sels d'addition avec un acide sont particulièrement préférés.

Parmi les para-aminophénols utilisables à titre de bases d'oxydation dans les compositions tinctoriales prêtes à l'emploi conformes à l'invention, on peut notamment citer les composés répondant à la formule (V) suivante, et leurs sels d'addition avec un acide : 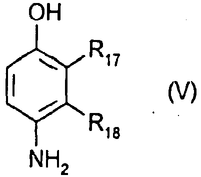 dans laquelle :
R₁₇ représente un atome d'hydrogène ou de fluor, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
R₁₈ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄), étant entendu qu'au moins un des radicaux R₁₇ ou R₁₈ représente un atome d'hydrogène.

Parmi les para-aminophénols de formule (V) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales prêtes à l'emploi conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales prêtes à l'emploi conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide. Parmi les dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triaminopyrimidine, et leurs sels d'addition avec un acide.
Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole, le 4,5-diamino 1-(4'-chlorobenzyl) pyrazole, et leurs sels d'addition avec un acide.

Parmi les méta-aminophénols de formule (I) utilisables à titre de coupleur dans les compositions tinctoriales prêtes à l'emploi conformes à l'invention, on peut plus particulièrement citer le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(y-hydroxypropylamino) 2-méthyl phénol, et leurs sels d'addition avec un acide.

Les colorants directs cationiques de formule (II) utilisables dans les compositions tinctoriales prêtes à l'emploi conformes à l'invention, sont des composés connus et sont décrits par exemple dans les demandes de brevets WO 95/01772, WO 95/15144 et EP-A-0 714 954. Parmi les colorants directs cationiques de formule (II) utilisables dans les compositions tinctoriales prêtes à l'emploi conformes à l'invention, on peut plus particulièrement citer les composés répondant aux structures (II) suivantes :

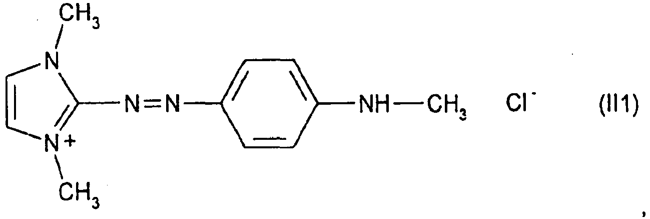

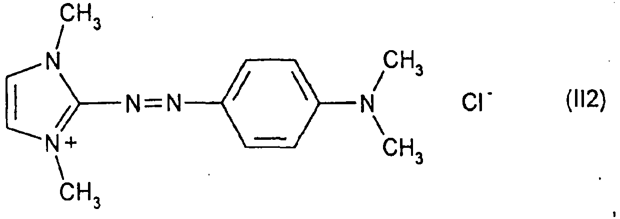

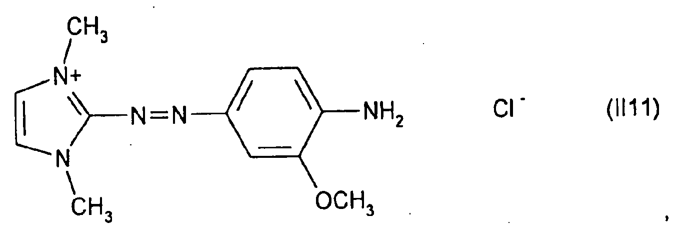

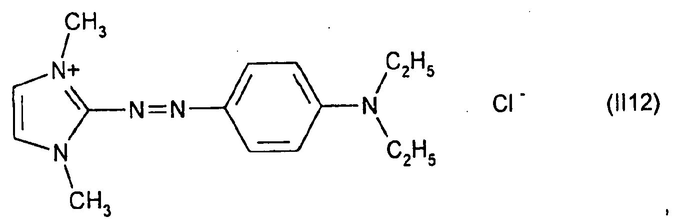

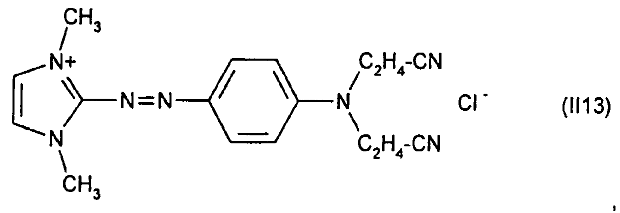

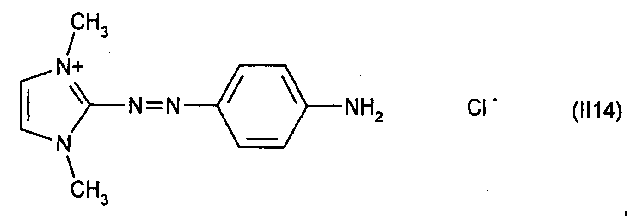

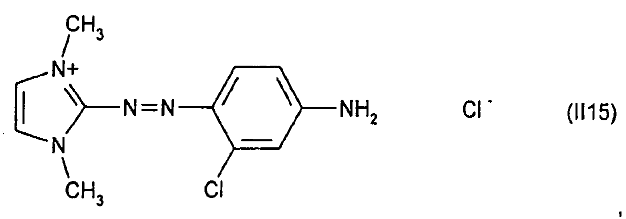

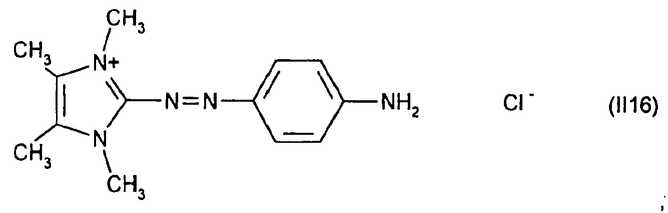

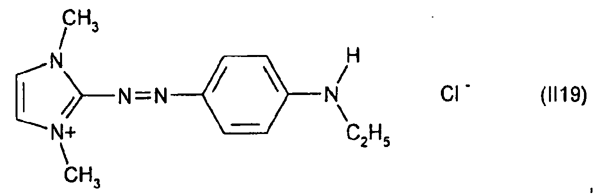

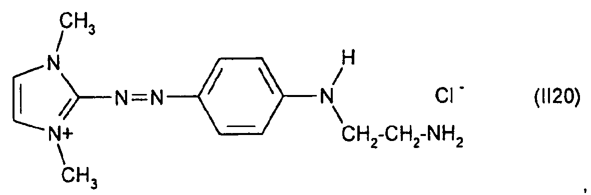

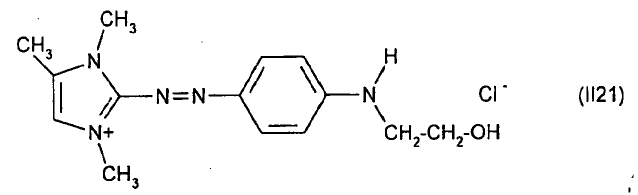

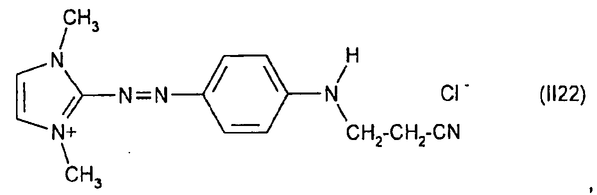

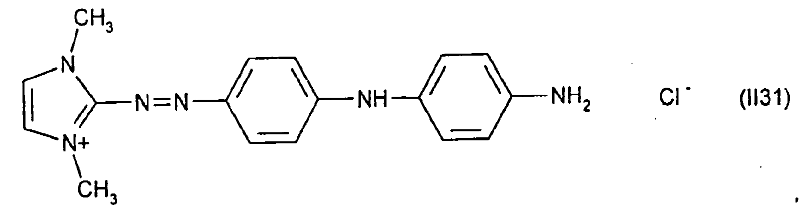

Parmi les composés décrits ci-dessus, on préfère tout particulièrement les composés répondant aux structures (II1), (II2), (II14) et (II31).

Les sels d'addition avec un acide utilisables dans le cadre des compositions tinctoriales de l'invention sont notamment choisis parmi les chlorhydrates, les bromhydrates, les sulfates et les tartrates.

L'agent oxydant présent dans la composition tinctoriale est choisi parmi les agents oxydants classiquement utilisés en coloration d'oxydation et de préférence parmi le peroxyde d'hydrogène, le peroxyde d'urée, les bromates de métaux alcalins, les persels tels que les perborates et persulfates. Le peroxyde d'hydrogène est particulièrement préféré.

La ou les bases d'oxydation représentent de préférence de 0,0001 à 10 % en poids environ du poids total de la composition tinctoriale prête à l'emploi et encore plus préférentiellement de 0.001 à 5 % en poids environ de ce poids.

Le ou les méta-aminophénols de formule (I) tels que définis ci-dessus représentent de préférence de 0,0001 à 5 % en poids environ du poids total de la composition tinctoriale prête à l'emploi et encore plus préférentiellement de 0,005 à 3 % en poids environ de ce poids.

Le ou les colorants directs cationiques de formule (II) conformes à l'invention, représentent de préférence de 0,001 à 10 % en poids environ du poids total de la composition tinctoriale prête à l'emploi et encore plus préférentiellement de 0,05 à 2 % en poids environ de ce poids.

Le pH de la composition tinctoriale telle que définie précédemment est généralement compris entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemples, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants, on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (VI) suivante : 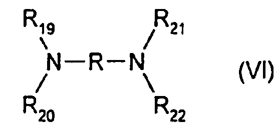 dans laquelle R est un reste propylène éventuellement substitué par un groupement hydroxyle ou un radical alkyle en C₁-C₄ ; R₁₉, R₂₀, R₂₁ et R₂₂, identiques ou différents, représentent un atome d'hydrogène, un radical alkyle en C₁-C₄ ou hydroxyalkyle en C₁-C₄.

La composition tinctoriale conforme à l'invention peut encore contenir, en plus des colorants définis ci-dessus, d'autres coupleurs et/ou d'autres colorants directs.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Les compositions tinctoriales prêtes à l'emploi conformes à l'invention peuvent également renfermer divers adjuvants utilisés classiquement dans les compositions pour la teinture des cheveux, tels que des agents tensio-actifs anioniques, cationiques, non-ioniques, amphotères ou leurs mélanges, des polymères anioniques, cationiques, non-ioniques, amphotères, ou leurs mélanges, des agents épaississants minéraux ou organiques, des agents antioxydants, des agents de pénétration, des agents séquestrants, des parfums, des tampons, des agents dispersants, des agents de conditionnement, des agents filmogènes, des agents conservateurs, des agents opacifiants.

Bien entendu, l'homme de l'art veillera à choisir le ou les éventuels composés complémentaires mentionnés ci-avant de manière telle que les propriétés avantageuses attachées intrinsèquement à la composition tinctoriale prête à l'emploi conforme à l'invention ne soient pas, ou substantiellement pas, altérées par la ou les adjonctions envisagées.

Les compositions tinctoriales prêtes à l'emploi conformes à l'invention peuvent se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres la composition tinctoriale prête à l'emploi telle que définie précédemment, et on laisse poser pendant 3 à 40 minutes environ, de préférence 5 à 30 minutes environ, après quoi on rince, on lave éventuellement au shampooing, on rince à nouveau et on sèche.

Selon une première forme de réalisation préférée, le procédé comporte une étape préliminaire consistant à stocker sous forme séparée, d'une part, une composition (A) comprenant, dans un milieu approprié pour la teinture, au moins une base d'oxydation, au moins un coupleur choisi parmi les méta-aminophénols de formule (I) telle que définie précédemment et au moins un colorant direct cationique choisi parmi les composés de formule (II) telle que définie précédemment et, d'autre part, une composition (B) renfermant, dans un milieu approprié pour la teinture, au moins un agent oxydant tel que défini précédemment, et à procéder à leur mélange au moment de l'emploi avant d'appliquer ce mélange sur les fibres kératiniques.

Selon une deuxième forme de réalisation préférée, le procédé comporte une étape préliminaire consistant à stocker sous forme séparée, d'une part, une composition (A) comprenant, dans un milieu approprié pour la teinture au moins une base d'oxydation, au moins un coupleur choisi parmi les méta-aminophénols de formule (I) telle que définie précédemment ; d'autre part une composition (A') comprenant, dans un milieu approprié pour la teinture, au moins un colorant direct cationique choisi parmi les composés de formule (II) telle que définie précédemment, et enfin, une composition (B) renfermant, dans un milieu approprié pour la teinture, au moins un agent oxydant tel que défini précédemment, et à procéder à leur mélange au moment de l'emploi avant d'appliquer ce mélange sur les fibres kératiniques.

La composition (A') utilisée selon cette deuxième variante du procédé conforme à l'invention, peut éventuellement se présenter sous forme de poudre, le ou les colorants directs cationiques de formule (II) conforme à l'invention constituant alors à lui (eux) seul(s) la totalité de ladite composition (A') ou étant éventuellement dispersé(s) dans un excipient pulvérulent organique et/ou minéral.

Lorsqu'il est présent dans la composition A', l'excipient organique peut être d'origine synthétique ou végétale et est choisi notamment parmi les polymères synthétiques réticulés et non réticulés, les polysaccharides comme les celluloses et les amidons modifiés ou non ainsi que les produits naturels les renfermant tels que la sciure de bois et les gommes végétales (guar, caroube, xanthane, etc...).

Lorsqu'il est présente dans la composition (A'), l'excipient minéral peut être constitué par des oxydes métalliques tels que les oxydes de titane, les oxydes d'aluminium, le kaolin, le talc, les silicates, le mica et les silices.
Un excipient avantageusement préféré selon l'invention est la sciure de bois.

La composition (A') en poudre peut encore renfermer des liants ou des produits d'enrobage dans une quantité ne dépassant pas de préférence 3% en poids environ du poids total de ladite composition (A').

Ces liants sont de préférence choisis parmi les huiles et les corps gras liquides d'origine minérale, synthétique, animale ou végétale.

La composition (A') peut éventuellement encore contenir d'autres adjuvants, à l'état de poudre, en particulier des tensio-actifs de toute nature, des agents de conditionnement du cheveu comme par exemple des polymères cationiques, etc...

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus, un second compartiment éventuel renferme la composition (A') telle que définie ci-dessus lorsqu'elle est présente et un troisième compartiment renferme la composition oxydante (B) telle que définie ci-dessus. Ces dispositifs peuvent être équipés d'un moyen permettant de délivrer sur les cheveux le mélange souhaité, tel que les dispositifs décrits dans le brevet FR-A-2 586 913 au nom de la demanderesse.

Les exemples qui suivent sont destinés à illustrer l'invention sans pour autant en limiter la portée.

### EXEMPLES

### EXEMPLES 1 à 4

On a préparé les compositions 1 (A) à 4 (A), conformes à l'invention, suivantes (teneurs en grammes) :

| **COMPOSITION** | **1 (A)** | **2 (A)** | **3 (A)** | **4 (A)** |
|---|---|---|---|---|
| | | | | |
| Paratoluylénediamine | 0,25 | - | - | - |
| Para-aminophénol | 0,30 | 0,50 | 0,15 | - |
| Paraphénylènediamine | - | 0,20 | - | 0,30 |
| | | | | |
| 5-N-(β-hydroxyéthyl)amino 2-méthyl phénol | 0,5 | 0,8 | 0,17 | - |
| 5-amino 2-méthyl phénol | - | - | - | 0,30 |
| | | | | |
| Colorant cationique de structure (II2) | 0,15 | - | - | |
| Colorant cationique de structure (II14) | - | 0,20 | 0,05 | - |
| Colorant cationique de structure (II1) | - | - | - | 0,1 |
| | | | | |
| Support de teinture commun (*) | (*) | (*) | (*) | (*) |
| Eau q.s.p. | 100 g | 100 g | 100 g | 100 g |

(*) support de teinture commun :

| | |
|---|---|
| - Alcool oléique polyglycérolé à 2 moles de glycérol | 4,0 g |
| - Alcool oléique polyglycérolé à 4 moles de glycérol, à 78 % de matières actives (M.A.) | 5,69 g M.A. |
| - Acide oléique | 3,0 g |
| - Amine oléique à 2 moles d'oxyde d'éthylène vendue sous la | |
| dénomination commerciale ETHOMEEN 012 par la société AKZO | 7,0 g |
| - Laurylamino succinamate de diéthylaminopropyle, sel de sodium, à 55 % de M.A. | 3,0 g M.A. |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse, à 35 % de M.A. | 0,455 g M.A. |
| - Acétate d'ammonium | 0,8 g |
| - Antioxydant, séquestrant | q.s. |
| - Parfum, conservateur | q.s. |
| - Ammoniaque à 20 % de NH₃ | 10,0 g |

Au moment de l'emploi, on a mélangé chacune de ces compositions 1 (A) à 4 (A) avec une quantité égale d'une composition (B) constituée par une solution de peroxyde d'hydrogène à 20 volumes (6 % en poids).

Chaque composition résultante (composition prête à l'emploi conforme à l'invention) a été appliquée pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes dans les nuances figurant dans le tableau ci-dessous :

| | |
|---|---|
| EXEMPLE [COMPOSITION] | NUANCE OBTENUE |
| 1 [1 (A)] | Blond foncé à reflet rouge intense |
| 2 [2 (A)] | Blond à reflet cuivré rouge intense |
| 3 [3 (A)] | Blond clair à reflet cuivré rouge |
| 4 [4 (A)] | Blond à reflet violine rouge |

Les nuances obtenues ont présenté une très bonne ténacité aux shampooings ultérieurs.

Selon une variante de l'invention, les colorants directs cationiques de structures (II2), (II14) et (II1) peuvent être incorporés dans les compositions colorantes 1 (A), 2 (A), 3 (A) et 4 (A) au moment de l'emploi.

### EXEMPLE 5

On a préparé la composition 5 (A) suivante :

| | |
|---|---|
| - 1,4-diamino benzène | 0,40 g |
| - 5-amino 2-méthyl phénol | 0,45 g |
| - Support de teinture commun tel que décrit précédemment pour les exemples 1 à 4 | (*) |
| - Eau déminéralisée q.s.p. | 100 g |

On a préparé la composition 5 (A') suivante :

| | |
|---|---|
| - Colorant cationique de structure (II2) | 4 g |
| - Polyammonium quaternaire vendu sous la dénomination commerciale CELQUAT SC-240 par la société National Starch | 10 g |
| - Sciure de bois q.s.p. | 100 g |

Au moment de l'emploi, on a mélangé une partie en poids de la composition 5 (A) ci-dessus avec 0,1 partie en poids de la composition 5 (A') et avec une partie en poids d'une composition (B) constituée par une solution de peroxyde d'hydrogène à 20 volumes (6 % en poids).

La composition résultante a été appliquée pendant 30 minutes sur des mèches de cheveux gris naturels à 90 % de blancs. Les cheveux ont ensuite été rincées, lavées avec un shampooing standard puis séchées.

Les cheveux ont été teints dans une nuance châtain clair à reflet rouge intense résistant très bien aux shampooings ultérieurs.

## Revendications

1. Composition prête à l'emploi pour la teinture d'oxydation des fibres kératiniques et en particulier des fibres kératiniques humaines telles que les cheveux **caractérisée par le fait qu'**elle comprend, dans un milieu approprié pour la teinture :
- au moins une base d'oxydation,
- au moins un coupleur choisi parmi les méta-aminophénols de formule (I) suivante et leurs sels d'addition avec un acide : 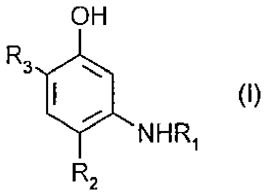 dans laquelle :
- R₁ représente un atome d'hydrogène, un radical alkyle en C₁-C₄, monohydroxyalkyle en C₁-C₄ ou polyhydroxyalkyle en C₂-C₄,
- R₂ représente un atome d'hydrogène, un radical alkyle en C₁-C₄, alcoxy en C₁-C₄ ou un atome d'halogène choisi parmi le chlore, le brome ou le fluor,
- R₃ représente un radical alkyle en C₁-C₄, alcoxy en C₁-C₄, monohydroxyalkyle en C₁-C₄, polyhydroxyalkyle en C₂-C₄, monohydroxyalcoxy en C₁-C₄ ou polyhydroxyalcoxy en C₂-C₄,
- au moins un colorant direct cationique choisi parmi les composés de formule (II) suivante : 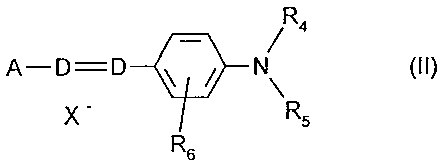
dans laquelle :
D représente un atome d'azote ou le groupement -CH,
R₄ et R₅, identiques ou différents, représentent un atome d'hydrogène ; un radical alkyle en C₁-C₄ pouvant être substitué par un radical -CN, -OH ou - NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₆ représente un atome d'hydrogène ou d'halogène choisi parmi le chlore, le brome, l'iode et le fluor, un radical alcoxy en C₁-C₄ ou acétyloxy,
X - représente un anion de préférence choisi parmi le chlorure, le méthyl sulfate et l'acétate,
A représente un groupement de structures A₁, A₇ et A₁₉: 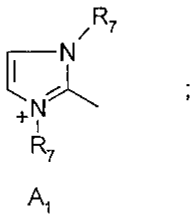 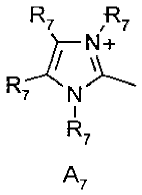 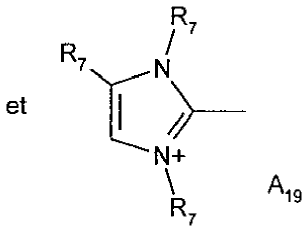 dans lesquelles R₇ représente un radical alkyle en C₁-C₄ pouvant être substitué par un radical hydroxyle; et
- au moins un agent oxydant.

2. Composition selon la revendication 1, **caractérisée par le fait que** la ou les bases d'oxydation sont choisies parmi les paraphénylènediamines, les bis-phénylalkylènediamines, les para-aminophénols, les ortho aminophénols et les bases d'oxydation hétérocycliques.

3. Composition selon la revendication 2, **caractérisée par le fait que** la ou les bases d'oxydation sont choisies parmi les paraphénylènediamines et les para-aminophénols.

4. Composition selon la revendication 2 ou 3, **caractérisée par le fait que** les paraphénylènediamines sont choisies parmi les composés de formule (III) suivante et leurs sels d'addition avec un acide : 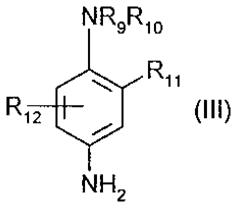 dans laquelle :
R₉ représente un atome d'hydrogène, un radical alkyle en C₁-C₄, monohydroxyalkyle en C₁-C₄, polyhydroxyalkyle en C₂-C₄, phényle, 4'-aminophényle ou alcoxy(C₁-C₄)alkyle en C₁-C₄,
R₁₀ représente un atome d'hydrogène, un radical alkyle en C₁-C₄, monohydroxyalkyle en C₁-C₄ ou polyhydroxyalkyle en C₂-C₄,
R₁₁ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
R₁₂ représente un atome d'hydrogène ou un radical alkyle en C₁-C₄.

5. Composition selon la revendication 4, **caractérisée par le fait que** les paraphénylènediamines de formule (III) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihy-droxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, et leurs sels d'addition avec un acide.

6. Composition selon la revendication 2, **caractérisée par le fait que** les bis-phénylalkylènediamines sont choisies parmi les composés de formule (IV) suivante, et leurs sels d'addition avec un acide : 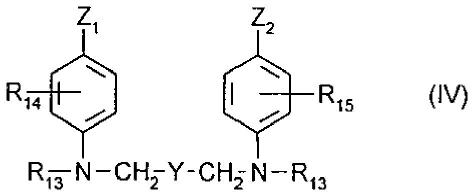 dans laquelle :
Z₁ et Z₂, identiques ou différents, représentent un radical hydroxyle ou NHR₁₆ dans lequel R₁₆ représente un atome d'hydrogène ou un radical alkyle en C₁-C₄,
R₁₃ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₁₄ et R₁₅, identiques ou différents, représentent un atome d'hydrogène ou d'halogène ou un radical alkyle en C₁-C₄,
Y représente un radical pris dans le groupe constitué par les radicaux suivants :
-(CH₂)ₙ ; -(CH₂)ₘ-O-(CH₂)ₘ; -(CH₂)ₘ-CHOH-(CH₂)ₘ et 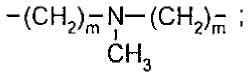
dans lesquels n est un nombre entier compris entre 0 et 8 inclusivement et m est un nombre entier compris entre 0 et 4 inclusivement.

7. Composition selon la revendication 6, **caractérisée par le fait que** les bis-phénylalkylènediamines de formules (IV) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl aminophényl) tétraméthylènediamine, la N,N'-bis-(éthyl) N,N'-bis-(4'-amino, 3'-méthylphényl) éthylènediamine, et leurs sels d'addition avec un acide.

8. Composition selon la revendication 2 ou 3, **caractérisée par le fait que** les para-aminophénols sont choisis parmi les composés de formule (V) suivante, et leurs sels d'addition avec un acide : 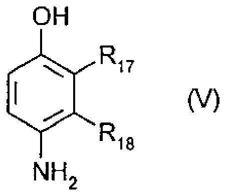 dans laquelle :
R₁₇ représente un atome d'hydrogène ou de fluor, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
R₁₈ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄), étant entendu qu'au moins un des radicaux R₁₇ ou R₁₈ représente un atome d'hydrogène.

9. Composition selon la revendication 8, **caractérisée par le fait que** les para-aminophénols de formule (V) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

10. Composition selon la revendication 2, **caractérisée par le fait que** les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

11. Composition selon la revendication 2, **caractérisée par le fait que** les bases hétérocycliques sont choisies parmi les dérivés pyridiniques, les dérivés pyrimidiniques, les dérivés pyrazoliques, et leurs sels d'addition avec un acide.

12. Composition selon l'une quelconque des revendications précédentes, **caractérisée par le fait que** les méta-amino-phénols de formule (I) sont choisis parmi le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, et leurs sels d'addition avec un acide.

13. Composition selon l'une quelconque des revendications précédentes, **caractérisée par le fait que** les colorants directs cationiques de formule (II) sont choisis parmi les composés répondant aux structures (II) suivantes

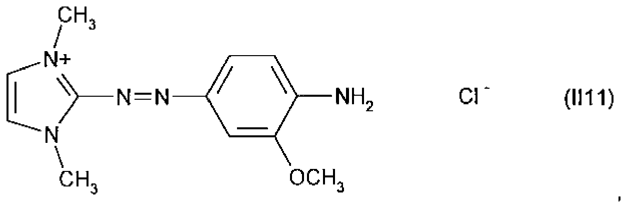

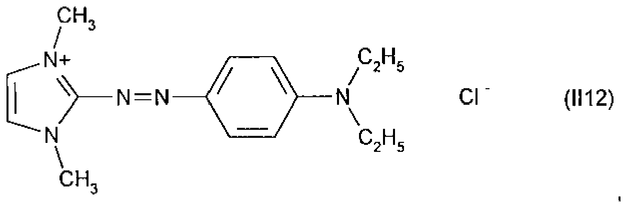

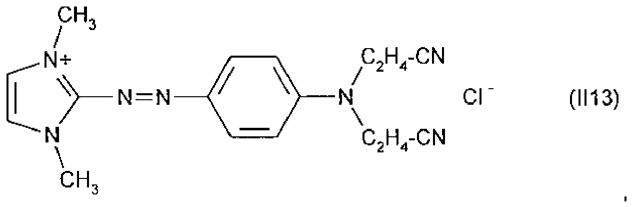

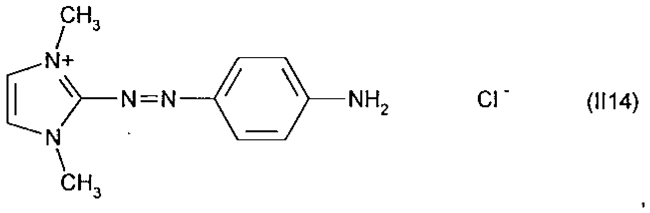

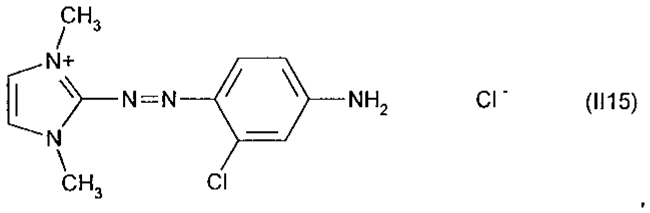

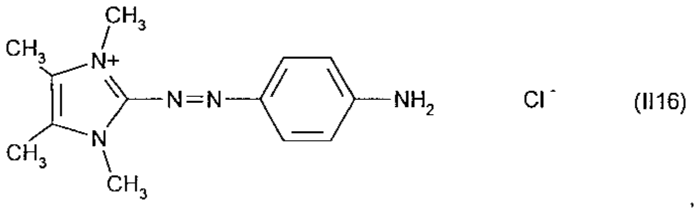

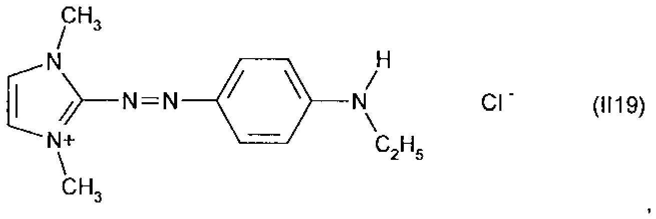

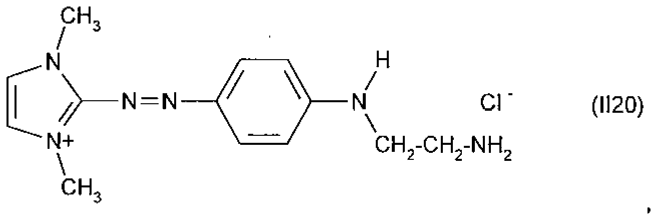

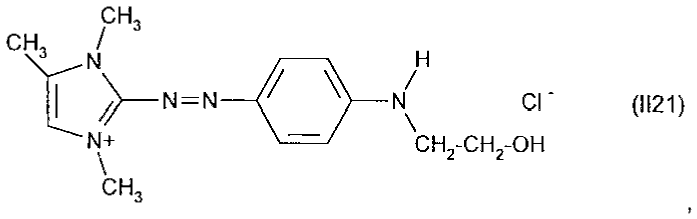

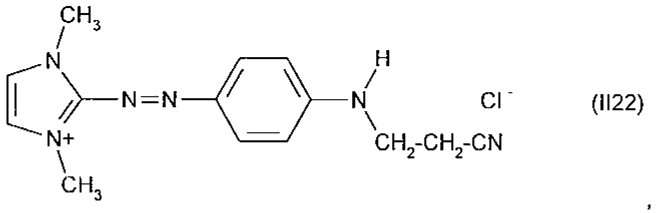

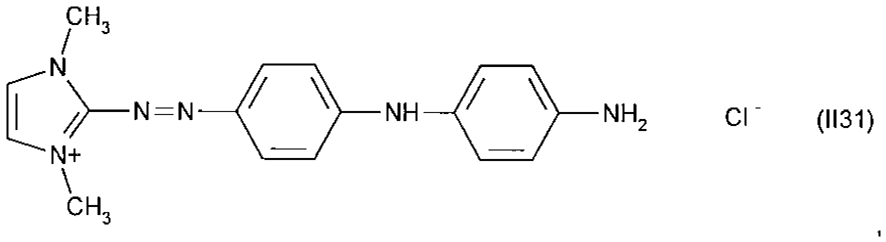

14. Composition selon la revendication 13, **caractérisée par le fait que** les colorants directs cationiques de formule (II) sont choisis parmi les composés répondant aux structures (II1), (II2), (II14) et (II31).

15. Composition selon l'une quelconque des revendications précédentes, **caractérisée par le fait que** les sels d'addition avec un acide sont choisis parmi les chlorhydrates, les bromhydrates, les sulfates et les tartrates.

16. Composition selon l'une quelconque des revendications précédentes, **caractérisée par le fait que** l'agent oxydant est choisi parmi le peroxyde d'hydrogène, le peroxyde d'urée, les bromates de métaux alcalins, les persels tels que les perborates et persulfates.

17. Composition selon l'une quelconque des revendications précédentes, **caractérisée par le fait que** la ou les bases d'oxydation représentent de 0,0001 à 10 % en poids du poids total de la composition tinctoriale prête à l'emploi.

18. Composition selon l'une quelconque des revendications précédentes, **caractérisée par le fait que** le ou les méta-aminophénols de formule (I) représentent de 0,0001 à 5 % en poids du poids total de la composition tinctoriale prête à l'emploi.

19. Composition selon l'une quelconque des revendications précédentes, **caractérisée par le fait que** le ou les colorants directs cationiques de formule (II) représentent de 0,001 à 10 % en poids du poids total de la composition tinctoriale prête à l'emploi.

20. Composition selon l'une quelconque des revendications précédentes, **caractérisée par le fait qu'**elle présente un pH compris entre 5 et 12.

21. Composition selon l'une quelconque des revendications précédentes, **caractérisée par le fait que** le milieu approprié pour la teinture est constitué par de l'eau ou par un mélange d'eau et d'au moins un solvant organique.

22. Procédé de teinture des fibres kératiniques et en particulier des fibres kératiniques humaines telles que les cheveux **caractérisé par le fait que** l'on applique sur ces fibres au moins une composition tinctoriale telle que définie dans l'une quelconque des revendications 1 à 21.

23. Procédé selon la revendication 22, **caractérisé par le fait qu'**il comporte une étape préliminaire consistant à stocker sous forme séparée, d'une part, une composition (A) comprenant, dans un milieu approprié pour la teinture, au moins une base d'oxydation, au moins un coupleur choisi parmi les méta-aminophénols de formule (I) tels que définis dans la revendication 1 ou 12, et au moins un colorant direct cationique choisi parmi les composés de formule (II) tels que définis dans la revendication 1, 13 ou 14, et d'autre part, une composition (B) renfermant, dans un milieu approprié pour la teinture, au moins un agent oxydant, et à procéder à leur mélange au moment de l'emploi avant d'appliquer ce mélange sur les fibres kératiniques.

24. Procédé de teinture selon la revendication 22, **caractérisé par le fait qu'**il comporte une étape préliminaire consistant à stocker sous forme séparée, d'une part, une composition (A) comprenant, dans un milieu approprié pour la teinture au moins une base d'oxydation, au moins un coupleur choisi parmi les méta-aminophénols de formule (I) tels que définis dans la revendication 1 ou 12 ; d'autre part une composition (A') comprenant, dans un milieu approprié pour la teinture, au moins un colorant direct cationique choisi parmi les composés de formule (II) tels que définis dans la revendication 1, 13 ou 14 ; et enfin, une composition (B) renfermant, dans un milieu approprié pour la teinture, au moins un agent oxydant et à procéder à leur mélange au moment de l'emploi avant d'appliquer ce mélange sur les fibres kératiniques.

25. Procédé selon la revendication 24, **caractérisé par le fait que** la composition (A') se présente sous forme de poudre.

26. Dispositif à plusieurs compartiments ou "kit" de teinture, **caractérisé par le fait qu'**un premier compartiment renferme la composition (A) telle que définie à la revendication 23 et un second compartiment renferme une composition oxydante (B).

27. Dispositif à plusieurs compartiments ou "kit" de teinture, **caractérisé par le fait qu'**un premier compartiment renferme une composition A telle que définie à la revendication 24, un second compartiment referme une composition A' telle que définie à la revendication 24 ou 25 et un troisième compartiment renferme une composition oxydante (B).

## Patentansprüche

1. Gebrauchsfertige Zusammensetzung zum oxidativen Färben von Keratinfasern und insbesondere menschlichen Keratinfasern, wie dem Haar, **dadurch gekennzeichnet, dass** sie in einem zum Färben geeigneten Medium enthält:
- mindestens eine Oxidationsbase,
- mindestens einen Kuppler, der unter den m-Aminophenolen der folgenden Formel (I) und den Additionssalzen dieser Verbindungen mit einer Säure ausgewählt ist: 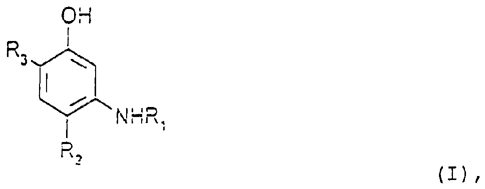 worin bedeuten:
- R₁ Wasserstoff, C₁₋₄-Alkyl, C₁₋₄-Monohydroxyalkyl oder C₂₋₄-Polyhydroxyalkyl,
- R₂ Wasserstoff, C₁₋₄-Alkyl, C₁₋₄-Alkoxy oder Halogen, das unter Chlor, Brom oder Fluor ausgewählt ist,
und
- R₃ C₁₋₄-Alkyl, C₁₋₄-Alkoxy, C₁₋₄-Monohydroxyalkyl, C₂₋₄-Polyhydroxyalkyl, C₁₋₄-Monohydroxyalkoxy oder C₂₋₄-Polyhydroxyalkoxy,
- mindestens einen kationischen Direktfarbstoff, der unter den Verbindungen der folgenden Formel (II) ausgewählt ist: 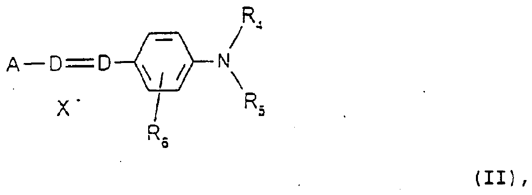 worin bedeuten:
- D ein Stickstoffatom oder die Gruppe -CH;
- R₄ und R₅, die identisch oder voneinander verschieden sind, Wasserstoff, eine C₁₋₄-Alkylgruppe, die mit einer Gruppe -CN, -OH oder -NH2 substituiert sein kann, oder 4'-Aminophenyl, oder R₄ und R₅ bilden mit einem Kohlenstoffatom des Benzolrings einen gegebenenfalls sauerstoffhaltigen oder stickstoffhaltigen Heterocyclus, der mit einer oder mehreren C₁₋₄-Alkylgruppen substituiert sein kann;
- R₆ Wasserstoff, Halogen, das unter Chlor, Brom, Iod und Fluor ausgewählt ist, C₁₋₄-Alkoxy oder Acetyloxy;
- X⁻ ein Anion, das vorzugsweise unter Chlorid, Methylsulfat und Acetat ausgewählt ist,
und
- A eine Gruppe mit den Strukturen A₁, A₇ und A₁₉: 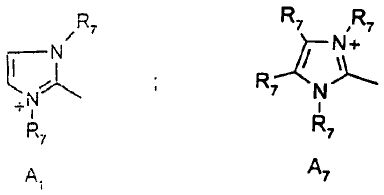 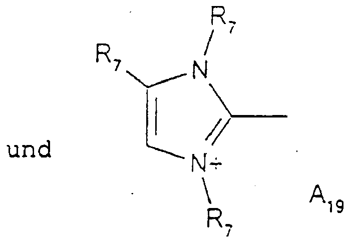 worin R₇ eine C₁₋₄-Alkylgruppe, die mit einer Hydroxygruppe substituiert sein kann, bedeutet und
- mindestens ein Oxidationsmittel.

2. Zusammensetzung nach Anspruch 1, **dadurch gekennzeichnet, dass** die Oxidationsbase oder die Oxidationsbasen unter p-Phenylendiaminen, Bisphenylalkylendiaminen, p-Aminophenolen, o-Aminophenolen und heterocyclischen Oxidationsbasen ausgewählt sind.

3. Zusammensetzung nach Anspruch 2, **dadurch gekennzeichnet, dass** die Oxidationsbase oder die Oxidationsbasen unter p-Phenylendiaminen und p-Aminophenolen ausgewählt sind.

4. Zusammensetzung nach Anspruch 2 oder 3, **dadurch gekennzeichnet, dass** die p-Phenylendiamine unter den Verbindungen der folgenden Formel (III) und den Additionssalzen dieser Verbindungen mit einer Säure ausgewählt sind: 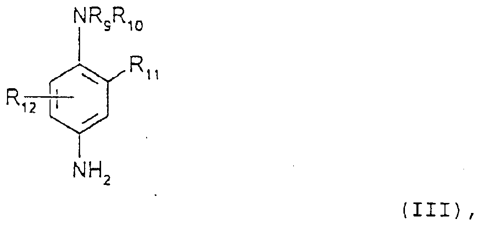 worin bedeuten:
- R₉ Wasserstoff, C₁₋₄-Alkyl, C₁₋₄-Monohydroxyalkyl, C₂₋₄-Polyhydroxyalkyl, Phenyl, 4'-Aminophenyl oder C₁₋₄-Alkoxy-C₁₋₄-Alkyl,
- R₁₀ Wasserstoff, C₁₋₄-Alkyl, C₁₋₄-Monohydroxyalkyl oder C₂₋₄-Polyhydroxyalkyl,
- R₁₁ Wasserstoff, Halogen, wie beispielsweise Chlor, Brom, Iod oder Fluor, C₁₋₄-Alkyl, C₁₋₄-Monohydroxyalkyl, C₁₋₄-Hydroxyalkoxy, C₁₋₄-Acetylaminoalkoxy, C₁₋₄-Mesylaminoalkoxy oder C₁₄-Carbamoylaminoalkoxy und
- R₁₂ Wasserstoff oder C₁₋₄-Alkyl.

5. Zusammensetzung nach Anspruch 4, **dadurch gekennzeichnet, dass** die p-Phenylendiamine der Formel (III) ausgewählt sind unter: p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methylanilin, N,N-Bis(β-hydroxyethyl)-p-phenylendiamin, 4-Amino-N,N-bis(β-hydroxyethyl)-3-methylanilin, 4-Amino-3-chlor-N,N-bis(β-hydroxyethyl)-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-p-phenylendiamin und den Additionssalzen dieser Verbindungen mit einer Säure.

6. Zusammensetzung nach Anspruch 2, **dadurch gekennzeichnet, dass** die Bisphenylalkylendiamine unter den Verbindungen der folgenden Formel (IV) und den Additionssalzen dieser Verbindungen mit einer Säure ausgewählt sind: 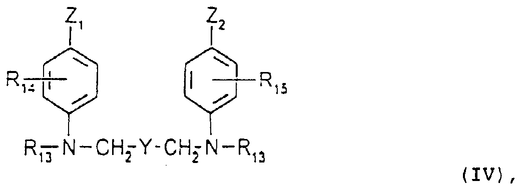 worin bedeuten:
- Z₁ und Z₂, die identisch oder voneinander verschieden sind, eine Hydroxygruppe oder NHR₁₆, worin R₁₆ Wasserstoff oder C₁₋₄-Alkyl bedeutet,
- R₁₃ Wasserstoff_{,} C₁₋₄-Alkyl, C₁₋₄-Monohydroxyalkyl, C₂₋₄-Polyhydroxyalkyl oder C₁₋₄-Aminoalkyl, worin der Aminorest substituiert sein kann,
- R₁₄ und R₁₅, die identisch oder voneinander verschieden sind, Wasserstoff, Halogen oder C₁₋₄-Alkyl,
und
- Y eine Gruppe, die unter den folgenden Gruppen ausgewählt ist:
-(CH₂)ₙ; -(CH₂)ₘ-O-(CH₂)ₘ; -(CH₂)ₘ-CHOH-(CH₂)ₘ und 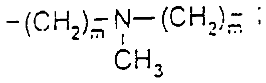
worin n null oder eine ganze Zahl im Bereich von einschließlich 1 bis 8 und m null oder eine ganze Zahl im Bereich von einschließlich 1 bis 4 bedeuten.

7. Zusammensetzung nach Anspruch 6, **dadurch gekennzeichnet, dass** die Bisphenylalkylendiamine der Formel (IV) ausgewählt sind unter: N,N'-Bis(β-hydroxyethyl)-N,N'-bis(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis(4'-amino-3'-methylphenyl)-ethylendiamin und den Additionssalzen dieser Verbindungen mit einer Säure.

8. Zusammensetzung nach Anspruch 2 oder 3, **dadurch gekennzeichnet, dass** die p-Aminophenole unter den Verbindungen der folgenden Formel (V) und den Additionssalzen dieser Verbindungen mit einer Säure ausgewählt sind: 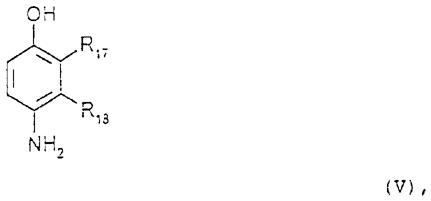 worin bedeuten:
- R₁₇ Wasserstoff, Fluor, C₁₋₄-Alkyl, C₁₋₄-Monohydroxyalkyl, C₁₋₄-Alkoxy-C₁₋₄-Alkyl, C₁₋₄-Aminoalkyl oder C₁₋₄-Hydroxyalkyl-C₁₋₄-Aminoalkyl und
- R₁₈ Wasserstoff, Fluor, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder C₁₋₄-Alkoxy-C₁₋₄-Alkyl,
mit der Maßgabe, dass mindestens eine der Gruppen R₁₇ oder R₁₈ Wasserstoff bedeutet.

9. Zusammensetzung nach Anspruch 8, **dadurch gekennzeichnet, dass** die p-Aminophenole der Formel (V) ausgewählt sind unter: p-Aminophenol, 4-Amino-3-methylphenol, 4-Amino-3-fluorphenol, 4-Amino-3-hydroxymethylphenol, 4-Amino-2-methylphenol, 4-Amino-2-hydroxymethylphenol, 4-Amino-2-methoxymethylphenol, 4-Amino-2-aminomethylphenol, 4-Amino-2-(β-hydroxyethylaminomethyl)-phenol, 4-Amino-2-fluorphenol und den Additionssalzen dieser Verbindungen mit einer Säure.

10. Zusammensetzung nach Anspruch 2, **dadurch gekennzeichnet, dass** die o-Aminophenole unter 2-Aminophenol, 2-Amino-5-methylphenol, 2-Amino-6-methylphenol, 5-Acetamido-2-aminophenol und den Additionssalzen dieser Verbindungen mit einer Säure ausgewählt sind.

11. Zusammensetzung nach Anspruch 2, **dadurch gekennzeichnet, dass** die heterocyclischen Basen unter Pyridinderivaten, Pyrimidinderivaten, Pyrazolderivaten und den Additionssalzen dieser Verbindungen mit einer Säure ausgewählt sind.

12. Zusammensetzung nach einem der vorhergehenden Ansprüche, **dadurch gekennzeichnet, dass** die m-Aminophenole der Formel (I) ausgewählt sind unter: 5-Amino-2-methoxyphenol, 5-Amino-2-(β-hydroxyethyloxy)-phenol, 5-Amino-2-methylphenol, 5-N-(β-hydroxyethyl)-amino-2-methylphenol, 5-N-(β-hydroxyethyl)-amino-4-methoxy-2-methylphenol, 5-Amino-4-methoxy-2-methylphenol, 5-Amino-4-chlor-2-methylphenol, 5-Amino-2,4 dimethoxyphenol, 5-(y-Hydroxypropylamino)-2-methylphenol und den Additionssalzen dieser Verbindungen mit einer Säure.

13. Zusammensetzung nach einem der vorhergehenden Ansprüche, **dadurch gekennzeichnet, dass** die kationischen Direktfarbstoffe der Formel (II) unter den Verbindungen der folgenden Strukturen (II) ausgewählt sind:

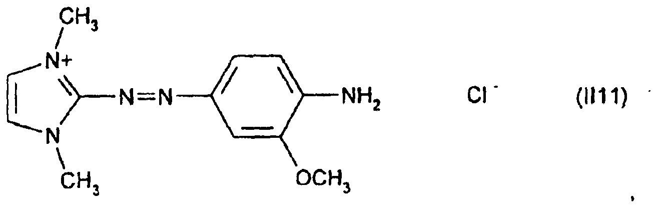

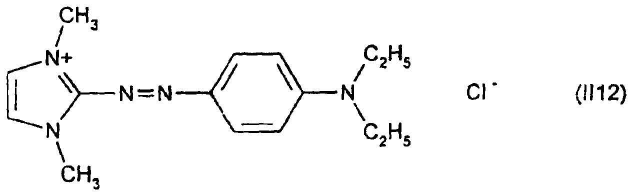

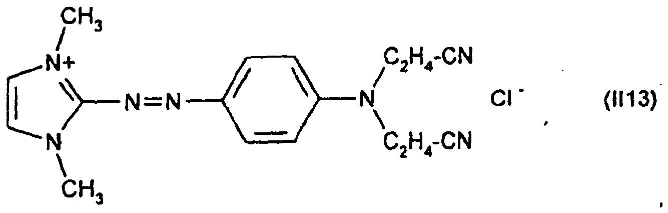

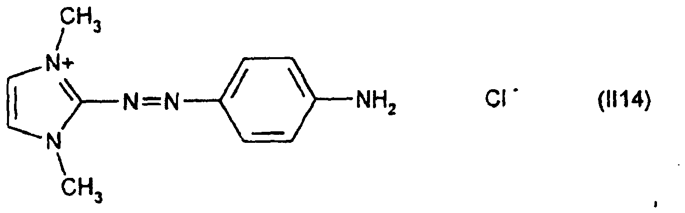

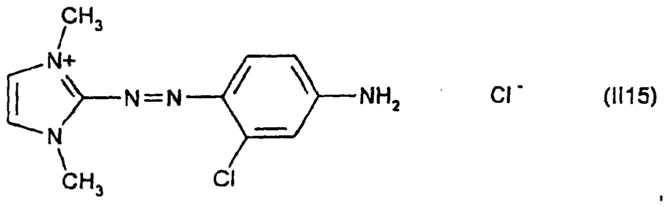

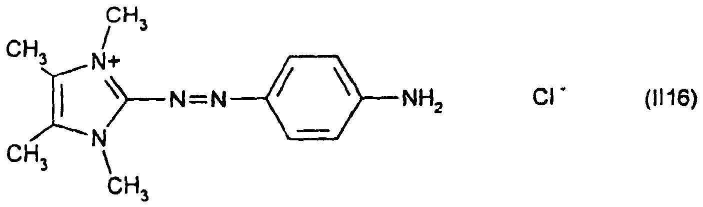

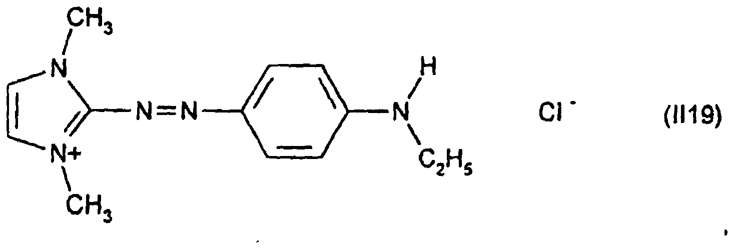

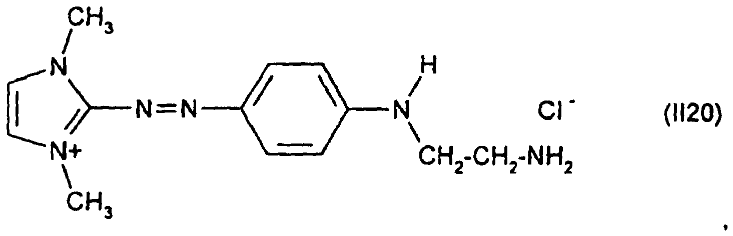

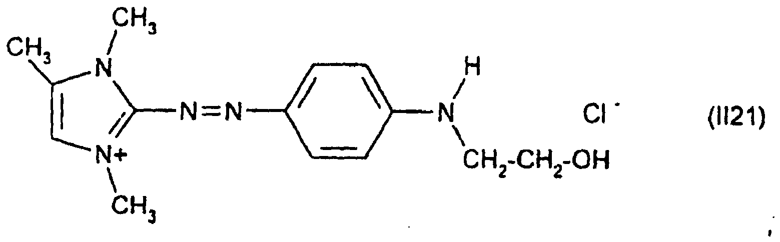

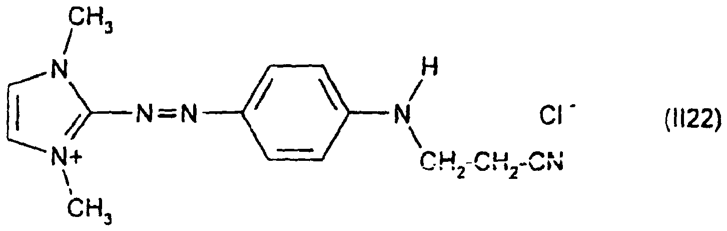

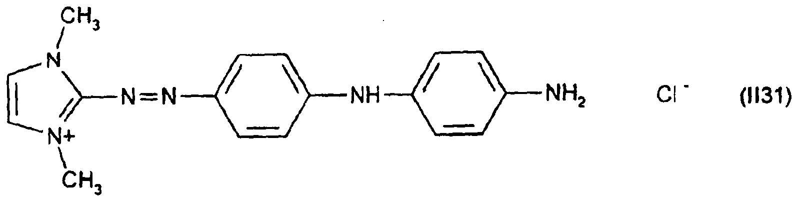

14. Zusammensetzung nach Anspruch 13, **dadurch gekennzeichnet, dass** die kationischen Direktfarbstoffe der Formel (II) unter den Verbindungen der Strukturen (II1), (II2), (II14) und (II31) ausgewählt sind.

15. Zusammensetzung nach einem der vorhergehenden Ansprüche, **dadurch gekennzeichnet, dass** die Additionssalze mit einer Säure unter Hydrochloriden, Hydrobromiden, Sulfaten und Tartraten ausgewählt sind.

16. Zusammensetzung nach einem der vorhergehenden Ansprüche, **dadurch gekennzeichnet, dass** das Oxidationsmittel unter Wasserstoffperoxid, Harnstoffperoxid, Alkalimetallbromaten und Salzen von Persäuren, wie Perboraten und Persulfaten, ausgewählt ist.

17. Zusammensetzung nach einem der vorhergehenden Ansprüche, **dadurch gekennzeichnet, dass** die Oxidationsbase oder die Oxidationsbasen 0,0001 bis 10 Gew.-% des Gesamtgewichts der gebrauchsfertigen Färbemittelzusammensetzung ausmachen.

18. Zusammensetzung nach einem der vorhergehenden Ansprüche, **dadurch gekennzeichnet, dass** das oder die m-Aminophenole der Formel (I) 0,0001 bis 5 Gew.-% des Gesamtgewichts der gebrauchsfertigen Färbemittelzusammensetzung ausmachen.

19. Zusammensetzung nach einem der vorhergehenden Ansprüche, **dadurch gekennzeichnet, dass** der oder die kationischen Direktfarbstoffe der Formel (II) 0,001 bis 10 Gew.-% des Gesamtgewichts der gebrauchsfertigen Färbemittelzusammensetzung ausmachen.

20. Zusammensetzung nach einem der vorhergehenden Ansprüche, **dadurch gekennzeichnet, dass** sie einen pH-Wert im Bereich von 5 bis 12 aufweist.

21. Zusammensetzung nach einem der vorhergehenden Ansprüche, **dadurch gekennzeichnet, dass** das zum Färben geeignete Medium aus Wasser oder einem Gemisch aus Wasser und mindestens einem organischen Lösungsmittel besteht.

22. Verfahren zum Färben von Keratinfasern und insbesondere menschlichen Keratinfasern, wie dem Haar, **dadurch gekennzeichnet, dass** auf die Fasern mindestens eine in einem der Ansprüche 1 bis 21 definierte Färbemittelzusammensetzung aufgebracht wird.

23. Verfahren nach Anspruch 22, **dadurch gekennzeichnet, dass** es einen vorbereitenden Schritt umfasst, der darin besteht, einerseits eine Zusammensetzung (A), die in einem zum Färben geeigneten Medium mindestens eine Oxidationsbase, mindestens einen Kuppler, der unter den in Anspruch 1 oder 12 definierten m-Aminophenolen der Formel (I) ausgewählt ist, und mindestens einen kationischen Direktfarbstoff, der unter den in Anspruch 1, 13 oder 14 definierten Verbindungen der Formel (II) ausgewählt ist, enthält, und andererseits eine Zusammensetzung (B), die in einem zum Färben geeigneten Medium mindestens ein Oxidationsmittel enthält, voneinander getrennt aufzubewahren und diese Zusammensetzungen bei der Anwendung zu vermischen, bevor das Gemisch auf die Keratinfasern aufgebracht wird.

24. Verfahren nach Anspruch 22, **dadurch gekennzeichnet, dass** es einen vorbereitenden Schritt umfasst, der darin besteht, einerseits eine Zusammensetzung (A), die in einem zum Färben geeigneten Medium mindestens eine Oxidationsbase und mindestens einen Kuppler, der unter den in Anspruch 1 oder 12 definierten m-Aminophenolen der Formel (I) ausgewählt ist, enthält, andererseits eine Zusammensetzung (A'), die in einem zum Färben geeigneten Medium mindestens einen kationischen Direktfarbstoff, der unter den in Anspruch 1, 13 oder 14 definierten Verbindungen der Formel (II) ausgewählt ist, enthält, und schließlich eine Zusammensetzung (B), die in einem zum Färben geeigneten Medium mindestens ein Oxidationsmittel enthält, voneinander getrennt aufzubewahren und diese Zusammensetzungen bei der Anwendung zu vermischen, bevor das Gemisch auf die Keratinfasern aufgebracht wird.

25. Verfahren nach Anspruch 24, **dadurch gekennzeichnet, dass** die Zusammensetzung (A') in Pulverform vorliegt.

26. Vorrichtung mit mehreren Abteilungen oder 'Kit' zum Färben, **dadurch gekennzeichnet, dass** eine erste Abteilung eine in Anspruch 23 definierte Zusammensetzung (A) und eine zweite Abteilung eine oxidierende Zusammensetzung (B) enthält.

27. Vorrichtung mit mehreren Abteilungen oder 'Kit' zum Färben, **dadurch gekennzeichnet, dass** eine erste Abteilung eine in Anspruch 24 definierte Zusammensetzung (A), eine zweite Abteilung eine in Anspruch 24 oder 25 definierte Zusammensetzung (A') und eine dritte Abteilung eine oxidierende Zusammensetzung (B) enthält.

## Claims

1. Ready-to-use composition for the oxidation dyeing of keratin fibres, and in particular human keratin fibres such as the hair, **characterized in that** it comprises, in a medium which is suitable for dyeing:
- at least one oxidation base,
- at least one coupler chosen from meta-aminophenols of formula (I) below, and the addition salts thereof with an acid: 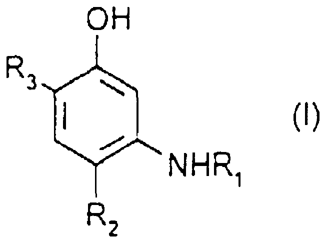 in which:
- R₁ represents a hydrogen atom or a C₁-C₄ alkyl, C₁-C₄ monohydroxyalkyl or C₂-C₄ polyhydroxyalkyl radical,
- R₂ represents a hydrogen atom, a C₁-C₄ alkyl or C₁-C₄ alkoxy radical or a halogen atom chosen from chlorine, bromine and fluorine,
- R₃ represents a C₁-C₄ alkyl, C₁-C₄ alkoxy, C₁-C₄ monohydroxyalkyl, C₂-C₄ polyhydroxyalkyl, C₁-C₄ mono-hydroxyalkoxy or C₂-C₄ polyhydroxyalkoxy radical,
- at least one cationic direct dye chosen from the compounds of formula (II) below: 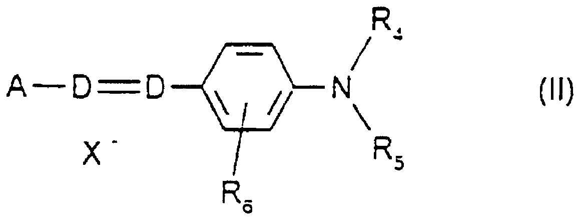 in which:
D represents a nitrogen atom or the -CH group,
R₄ and R₅, which may be identical or different,
represent a hydrogen atom; a C₁-C₄ alkyl radical which may be substituted with a -CN, -OH or -NH₂ radical or form, with a carbon atom of the benzene ring, a heterocycle which is optionally oxygenated or nitrogenous, which may be substituted with one or more C₁-C₄ alkyl radicals; a 4'-aminophenyl radical,
R₆ represents a hydrogen or halogen atom chosen from chlorine, bromine, iodine and fluorine, or a C₁-C₄ alkoxy or acetyloxy radical,
X⁻ represents an anion preferably chosen from chloride, methylsulfate and acetate,
A represents a group of structures A₁, A₇ and A₁₉: 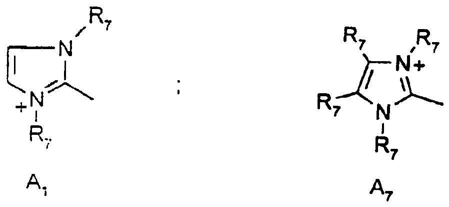 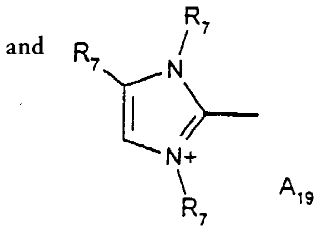
in which R₇ represents a C₁-C₄ alkyl radical which may be substituted with a hydroxyl radical; and
- at least one oxidizing agent.

2. Composition according to Claim 1, **characterized in that** the oxidation base(s) is(are) chosen from para-phenylenediamines, bis(phenyl)alkylenediamines, para-aminophenols, ortho-aminophenols and heterocyclic oxidation bases.

3. Composition according to Claim 2, **characterized in that** the oxidation base(s) is (are) chosen from para-phenylenediamines and para-aminophenols.

4. Composition according to Claim 2 or 3, **characterized in that** the para-phenylenediamines are chosen from the compounds of formula (III) below, and the addition salts thereof with an acid: 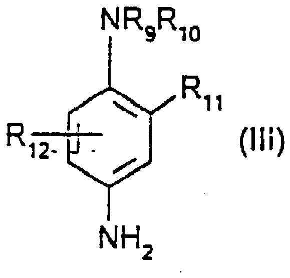 in which:
R₉ represents a hydrogen atom, a C₁-C₄ alkyl, C₁-C₄ monohydroxyalkyl, C₂-C₄ polyhydroxyalkyl, phenyl, 4'-aminophenyl or (C₁-C₄)alkoxy(C₁-C₄)alkyl radical, R₁₀ represents a hydrogen atom or a C₁-C₄ alkyl, C₁-C₄ monohydroxyalkyl or C₂-C₄ polyhydroxyalkyl radical,
R₁₁ represents a hydrogen atom, a halogen atom such as a chlorine, bromine, iodine or fluorine atom, or a C₁-C₄ alkyl, C₁-C₄ monohydroxyalkyl, C₁-C₄ hydroxyalkoxy, C₁-C₄ acetylaminoalkoxy, C₁-C₄ mesylaminoalkoxy or C₁-C₄ carbamoylaminoalkoxy radical,
R₁₂ represents a hydrogen atom or a C₁-C₄ alkyl radical.

5. Composition according to Claim 4, **characterized in that** the para-phenylenediamines of formula (III) are chosen from para-phenylenediamine, para-toluylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-amino-N,N-bis(β-hydroxyethyl)-3-methylaniline, 4-amino-3-chloro-N,N-bis(β-hydroxyethyl)aniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl-β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine and 2-β-acetylaminoethyloxy-para-phenylenediamine, and the addition salts thereof with an acid.

6. Composition according to Claim 2, **characterized in that** the bis(phenyl)alkylenediamines are chosen from the compounds of formula (IV) below, and the addition salts thereof with an acid: 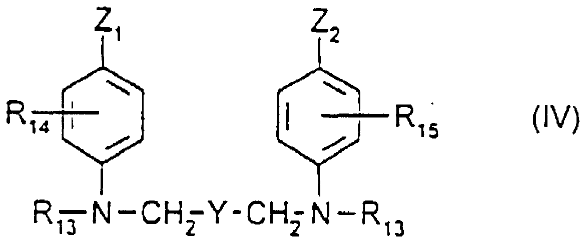 in which:
Z₁ and Z₂, which may be identical or different, represent a hydroxyl radical or NHR₁₆ in which R₁₆ represents a hydrogen atom or a C₁-C₄ alkyl radical,
R₁₃ represents a hydrogen atom or a C₁-C₄ alkyl, C₁-C₄ monohydroxyalkyl or C₂-C₄ polyhydroxyalkyl radical or a C₁-C₄ aminoalkyl radical in which the amino residue can be substituted,
R₁₄ and R₁₅, which may be identical or different, represent a hydrogen or halogen atom or a C₁-C₄ alkyl radical,
Y represents a radical taken from the group consisting of the following radicals:
- (CH₂)ₙ ; -(CH₂)ₘ-O-(CH₂)ₘ-(CH₂)ₘ-CHOH-(CH₂)ₘ and 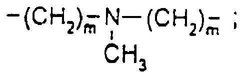
in which n is an integer between 0 and 8 inclusive and m is an integer between 0 and 4 inclusive.

7. Composition according to Claim 6, **characterized in that** the bis(phenyl)alkylenediamines of formula (IV) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)-ethylenediamine, N,N'-bis(4-aminophenyl)-tetramethylenediamine, N,N'-bis(β-hydroxyethyl)-N,N'-bis(4-aminophenyl)tetramethylenediamine, N,N'-bis(4-methylaminophenyl)tetramethylenediamine and N,N'-bis(ethyl)-N,N'-bis(4'-amino-3'-methylphenyl)-ethylenediamine, and the addition salts thereof with an acid.

8. Composition according to Claim 2 or 3, **characterized in that** the para-aminophenols are chosen from the compounds of formula (V) below, and the addition salts thereof with an acid: 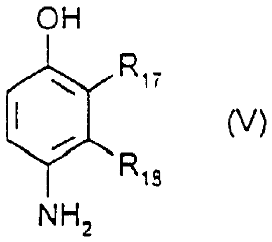 in which:
R₁₇ represents a hydrogen or fluorine atom or a C₁-C₄ alkyl, C₁-C₄ monohydroxyalkyl, (C₁-C₄)-alkoxy(C₁-C₄)alkyl, C₁-C₄ aminoalkyl or hydroxy(C₁-C₄)alkylamino-(C₁-C₄)alkyl radical,
R₁₈ represents a hydrogen or fluorine atom or a C₁-C₄-alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy-(C₁-C₄)alkyl radical,
it being understood that at least one of the radicals R₁₇ or R₁₈ represents a hydrogen atom.

9. Composition according to Claim 8, **characterized in that** the para-aminophenols of formula (V) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

10. Composition according to Claim 2, **characterized in that** the ortho-aminophenols are chosen from 2-aminophenol, 2-amino-5-methylphenol, 2-amino-6-methylphenol and 5-acetamido-2-aminophenol, and the addition salts thereof with an acid.

11. Composition according to Claim 2, **characterized in that** the heterocyclic bases are chosen from pyridine derivatives, pyrimidine derivatives and pyrazole derivatives, and the addition salts thereof with an acid.

12. Composition according to any one of the preceding claims, **characterized in that** the meta-aminophenols of formula (I) are chosen from 5-amino-2-methoxyphenol, 5-amino-2-(β-hydroxyethyloxy)phenol, 5-amino-2-methylphenol, 5-N-(β-hydroxyethyl)amino-2-methylphenol, 5-N-(β-hydroxyethyl)amino-4-methoxy-2-methylphenol, 5-amino-4-methoxy-2-methylphenol, 5-amino-4-chloro-2-methylphenol, 5-amino-2,4-dimethoxy-phenol and 5-(γ-hydroxypropylamino)-2-methylphenol, and the addition salts thereof with an acid.

13. Composition according to any one of the preceding claims, **characterized in that** the cationic direct dyes of formula (II) are chosen from the compounds corresponding to structures (II) below:

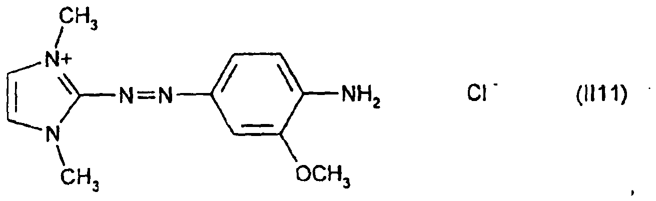

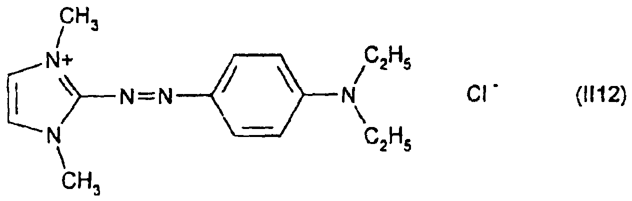

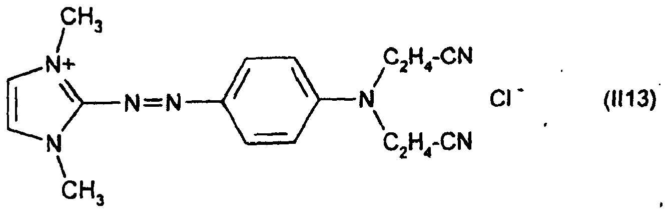

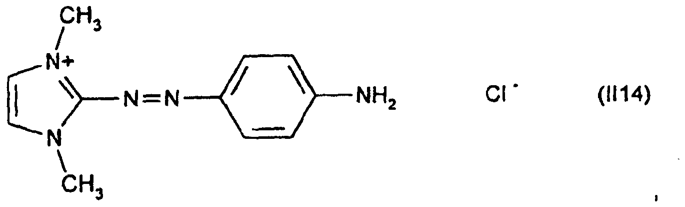

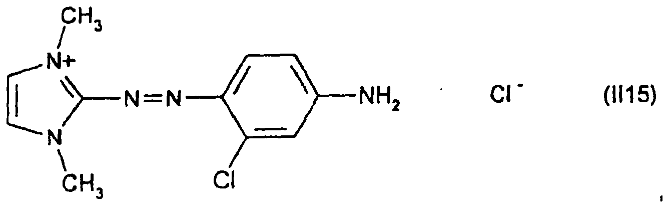

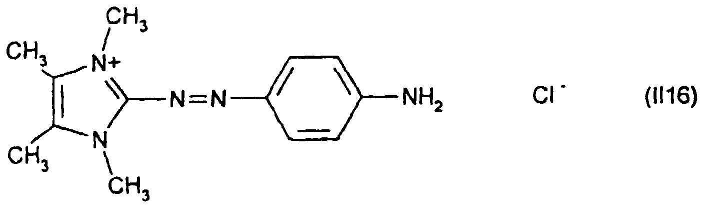

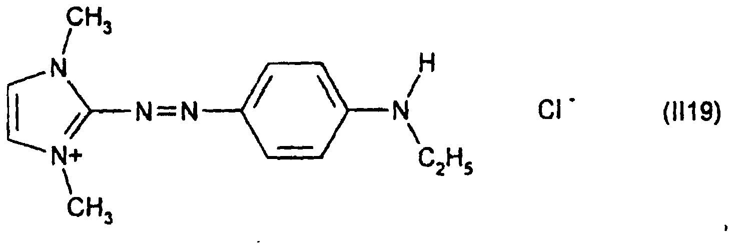

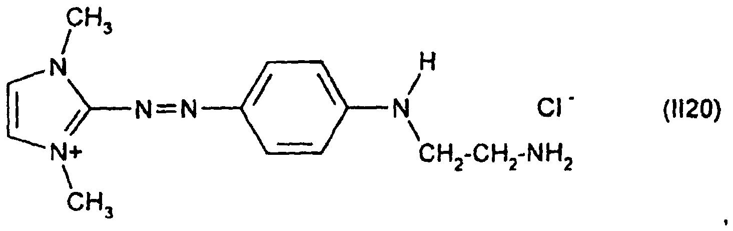

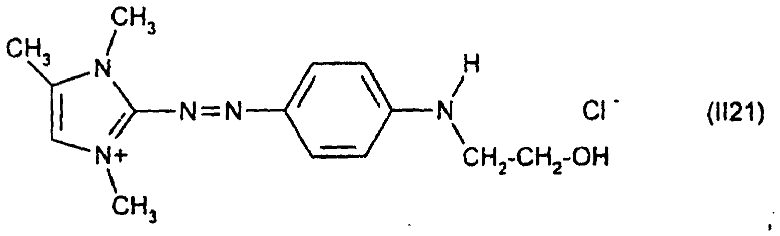

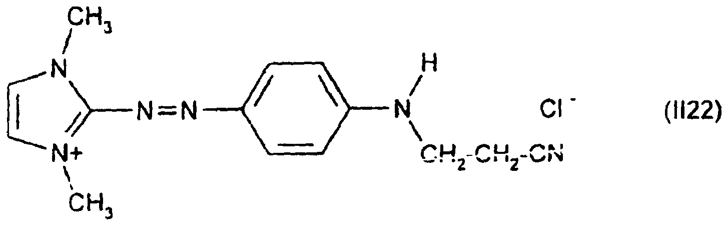

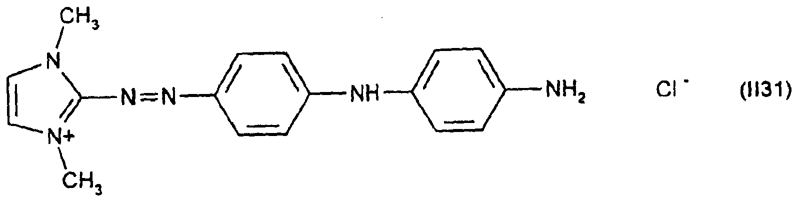

14. Composition according to Claim 13, **characterized in that** the cationic direct dyes of formula (II) are chosen from the compounds corresponding to structures (II1), (II2), (II14) and (II31).

15. Composition according to any one of the preceding claims, **characterized in that** the addition salts with an acid are chosen from the hydrochlorides, hydrobromides, sulfates and tartrates.

16. Composition according to any one of the preceding claims, **characterized in that** the oxidizing agent is chosen from hydrogen peroxide, urea peroxide, alkali metal bromates and persalts such as perborates and persulfates.

17. Composition according to any one of the preceding claims, **characterized in that** the oxidation base(s) represent(s) from 0.0001 to 10% by weight relative to the total weight of the ready-to-use dye composition.

18. Composition according to any one of the preceding claims, **characterized in that** the metaaminophenol(s) of formula (I) represent(s) from 0.0001 to 5% by weight relative to the total weight of the ready-to-use dye composition.

19. Composition according to any one of the preceding claims, **characterized in that** the cationic direct dye(s) of formula (II) represent(s) from 0.001 to 10% by weight relative to the total weight of the ready-to-use dye composition.

20. Composition according to any one of the preceding claims, **characterized in that** it has a pH of between 5 and 12.

21. Composition according to any one of the preceding claims, **characterized in that** the medium which is suitable for dyeing consists of water or of a mixture of water and at least one organic solvent.

22. Process for dyeing keratin fibres, and in particular human keratin fibres such as the hair, **characterized in that** at least one dye composition as defined in any one of Claims 1 to 21 is applied to these fibres.

23. Process according to Claim 22, **characterized in that** it includes a preliminary step which consists in separately storing, on the one hand, a composition (A) comprising, in a medium which is suitable for dyeing, at least one oxidation base, at least one coupler chosen from the meta-aminophenols of formula (I) as defined in Claim 1 or 12, and at least one cationic direct dye chosen from the compounds of formula (II) as defined in Claim 1, 13 or 14, and, on the other hand, a composition (B) containing, in a medium which is suitable for dyeing, at least one oxidizing agent, and in mixing them together at the time of use before applying this mixture to the keratin fibres.

24. Dyeing process according to Claim 22, **characterized in that** it includes a preliminary step which consists in separately storing, on the one hand, a composition (A) comprising, in a medium which is suitable for dyeing, at least one oxidation base, at least one coupler chosen from the meta-aminophenols of formula (I) as defined in Claim 1 or 12; on the other hand, a composition (A') comprising, in a medium which is suitable for dyeing, at least one cationic direct dye chosen from the compounds of formula (II) as defined in Claim 1, 13 or 14, and, lastly, a composition (B) containing, in a medium which is suitable for dyeing, at least one oxidizing agent, and in mixing them together at the time of use before applying this mixture to the keratin fibres.

25. Process according to Claim 24, **characterized in that** the composition (A') is in powder form.

26. Multi-compartment dyeing "kit" or device, **characterized in that** a first compartment contains the composition (A) as defined in Claim 23 and a second compartment contains an oxidizing composition (B).

27. Multi-compartment dyeing "kit" or device, **characterized in that** a first compartment contains a composition A as defined in Claim 24, a second compartment contains a composition A' as defined in Claim 24 or 25 and a third compartment contains an oxidizing composition (B).
